# EUROPEAN PATENT SPECIFICATION

(11) **EP 2 205 146 B1**
(45) Date of publication and mention of the grant of the patent: **20.04.2011**
(21) Application number: 08825888.4
(22) Date of filing: 23.10.2008
(51) Int. Cl.: A61B 5/00, A61B 5/0205, A61D 7/00, A61B 5/02, A61M 31/00

(54) **INFORMATION SYSTEM FOR ANIMALS**
INFORMATIONSSYSTEM FÜR TIERE
SYSTÈME D'INFORMATION POUR ANIMAUX

(30) Priority: 30.10.2007 NL 2000970
(43) Date of publication of application: 14.07.2010
(73) Proprietor: Agis Automatisering B.V., 3481 LT Harmelen (NL)
(72) Inventor: GRIFFIOEN, Gerard, Marie, NL-3481 LT Harmelen (NL)
(74) Representative: Klavers, Cornelis
(86) International application number: PCT/NL2008/050666
(87) International publication number: WO 2009/058004

(56) References cited:
- WO-A-03/065923
- WO-A-2005/020841
- WO-A-2007/004089
- FR-A- 2 801 491
- GB-A- 2 162 325
- US-A- 4 865 044
- US-A1- 2005 192 478
- US-B1- 6 253 871

## Description

The present invention relates to an information system, inter alia for use with animals, comprising a base station and a number of units being in wireless communication with said base station, which units are to be placed in the animal's ear and which are provided with one or more sensors for periodically measuring quantities such as temperature, sound, (ruminating) activity, movement, heart rate and/or biochemical values, at least in the ear, and which are connected to an activable transmitter for transmitting, upon activation of said transmitter, individual sensor measurement data to the base station.

The present invention also relates to an ear unit suitable for use in the information system.

As a result of the constantly increasing expansion in cattle farms, such as dairy farms, cattle farmers tend to concentrate more and more on managing their overall stock rather than individual animals, in particular cows, horses, pigs, sheep or the like. However, due attention for the animal's health and well-being is important both for the animal and for the cattle farmer, because healthy cows produce more milk and require lower health care costs. The use of antibiotics, for example, leads to higher costs, it affects the quality of the milk and the meat and increases the risk of resistance of the pathogen being fought therewith. A timely diagnosis will reduce the subsequently required use of antibiotics and the associated drawbacks.

A system used to delimited the claims from is known from WO 2005/020841. The known system furthermore discloses a temperature sensor provided on the ear unit.

The object of the present invention is to provide an improved information or monitoring system, by means of which an animal's illness or disorder can be established at an early stage, which illness or disorder can subsequently be remedied as soon as possible.

In order to accomplish that object, the animal information system according to the invention as defined in claim 1 is characterised in that the ear unit is provided with more than one sensor capable of measuring the temperature and disposed at two or more depths in the ear canal for measuring the same quantity at several depths in the ear.

The advantage of the information system according to the invention is that, depending on the adjustable period times, essential measurement data such as temperature, sound (for example of breathing), digestion, movement, heart rate and the like, can nevertheless be monitored on a practically continuous basis. One or more sensors measure different parameters or measurement data, which are usually transmitted in succession so as to reduce the need for momentary peak capacity. As a result of said continuous monitoring, it is possible to establish at an early stage that something, and even what exactly, is the matter with the animal, and that even before the disorder actually manifests itself in the form of visually perceptible characteristics or behaviour. Because it is possible to take action at that stage, the treatment of disorders that can be diagnosed in this way will be more effective and require less time, whilst in addition less medicine and/or antibiotics will be need. This is beneficial to the animal's well-being, and after the disorder - which will generally take a less severe course - the animal will sooner start to produce more milk again and regain its previous level.
Setting longer period times will lead to a reduced local consumption of the electrical power, which is usually obtained from batteries or from solar cells - that is needed in each ear unit. Periodically, every few minutes, for example every 5-10 minutes, the measurement data are transmitted to the base station via a sensor, so that the local memory capacity and the use thereof for the storage of the data need not be so large.

As a result of the use of an activable transmitter provided in or on the ear unit, the relatively high transmitting power needed for transmitting data, which may have been pre-analysed, only needs to be available when this is considered necessary, viz. upon activation of the transmitter. This reduces the local consumption of electric power even further, making it possible to use a power supply unit which is small and less heavy and inconvenient both for people and for animals.

One embodiment of the system according to the invention is characterised in that the ear unit is provided with a processor which is arranged for activating the transmitter when a measurement establishes the existence of individual sensor measurement data that fall outside a normal range.

Only if specific, continuously measured sensor measurement data or quantities derived therefrom appear to fall outside the normal range, will an alarm be raised, as it were, in this embodiment, and will the transmitter be activated to transmit the measurement data to the base station. Depending on the setting in the base station, the cattle farmer might for example receive an SMS message, informing him that an abnormality has been found to be present in one or more of the measured quantities or combinations thereof in the unfortunate animal identified by number 13. If necessary, direct action might even be taken, if desired, by stabling the animal in question and treating it.

The system according to the invention is characterised in that the ear unit is provided with more than one sensor, which sensors are disposed at different locations, for measuring the same quantity at several depths in the ear.

Surprisingly, it has been found that in particular the measurement of the temperature at different depths in the ear, and in particular the determination of the temperature difference and the changes in said difference, provides important options as regards analysis and diagnosis, which make it possible to determine the existence of a respective illness or disorder at a very early stage with a higher degree of accuracy. This makes it possible to start an adequate treatment even sooner. As a result, less pharmaceutical and other types of remedies will be needed, and that for a shorter period of time. Because of this, the cow's original milk production will be restored even sooner.

Is has been found that animals, in particular cows, gradually contract their blood vessels further upon sensing an approaching illness or disorder, which results in a difference between temperature values measured by sensors at different depths in the ear canal. Besides other factors, this temperature difference, or the changes in said temperature difference, forms an important lead for an analysis and diagnosis so as to arrive at a reliable determination of the illness or disorder in question.

In another embodiment of the invention, the base station and the ear units are connected to or provided with transceivers.

The advantage of this is that it is possible to obtain information regarding the current health condition of all the animals, or only of selected animals in the system, at the initiative of the base station. At the request of the base station, the transceivers will transmit the requested information.

One or more of the transceivers may be configured as a relaying unit, so that when an animal is far removed from the base station and its transmitter cannot reach said base station, the animal's data can be transmitted to the base station via one or more intermediate units functioning as a relaying unit, or vice versa.

A preferred embodiment of the system according to the invention is characterised in that the ear unit comprises a cavity which is open towards the ear canal or which is porous, which cavity can be filled with a medicinal and/or health-promoting substance.

The cavity may for example be filled with a gradually disintegrating, medicinally active substance, such as a pill, and/or with selenium, for example, and/or a vitamin, such as vitamin E. After the cavity in the ear unit, which may already be present in the ear, has been filled, the substance present therein is gradually delivered to the blood circulating in the blood vessels of the ear canal, referred to as "transdermal administration", via a porous portion. In this way a delayed or slow-release activity that may be desirable in the case of some disorders is obtained, thus giving treatment a round-the-clock nature. Some substances, such as vitamin E, appeared to be buffered in the skin, from which buffer the animal can draw when and if the need arises.

Another preferred embodiment is characterised in that the system is arranged for influencing the amount of substance being released in blood vessels of the ear canal from the base station, for example by regulating or opening one or more closed parts of the cavity.

By possibly configuring the cavity as a multi-part cavity, doses of different medicines can be administered simultaneously or in succession, whilst the animal can normally continue to walk around in the field or in the stable.

The present invention further relates to an ear unit suitable for use in the information system, which is characterised in that the ear unit has a certain degree of stiffness between the parts of the ear unit to be provided between helix and concha in the ear.

This construction of the ear unit ensures that the ear unit will be properly secured in place in the ear canal after insertion, so that it will not fly out when the animal suddenly moves or turns its head. The fact that the ear unit is properly secured in the ear canal and immovably abuts the walls thereof leads to a reliable and exact diagnosis and a better assessment of a possible illness or disorder.

A stiffness between shore 35 and 75, in particular between shore 50 and 70, more in particular around shore 60-65, of the part between helix and concha is to be preferred for the ear unit, which for the rest must be kept flexible and light in weight. Because of this, it is easy to insert and remove and not objectionable for the animal to wear.

The information system and the ear unit according to the present invention will now be explained in more detail with reference to the figures below, in which like parts are provided with the same numerals. In the drawing:
Figure 1 schematically shows a possible embodiment of the information system, for example for animals, according to the invention; and
Figure 2 is a schematic representation of two respective views of an ear unit according to the invention which is suitable for use in the information system of figure 1.

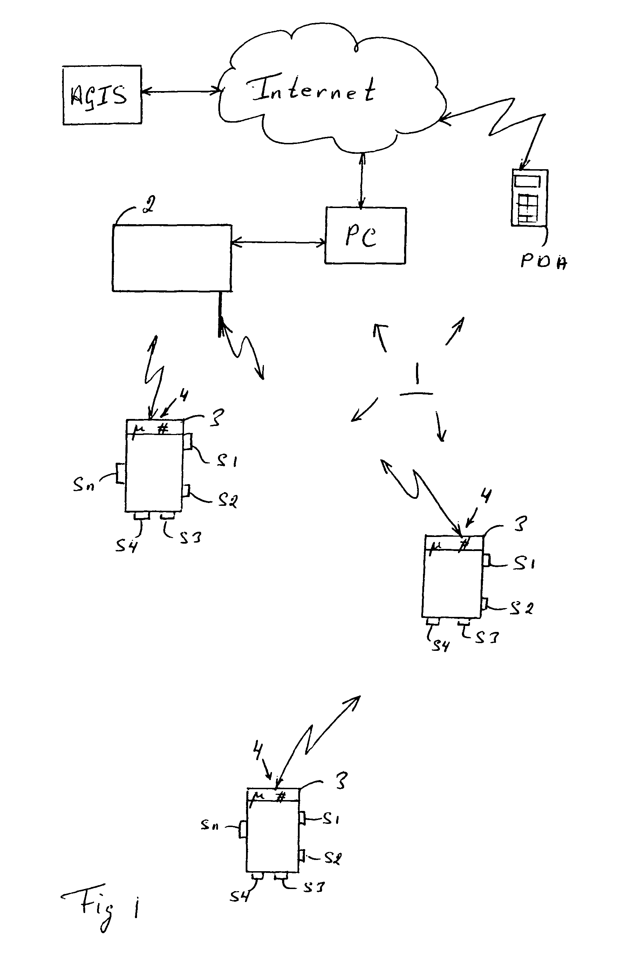

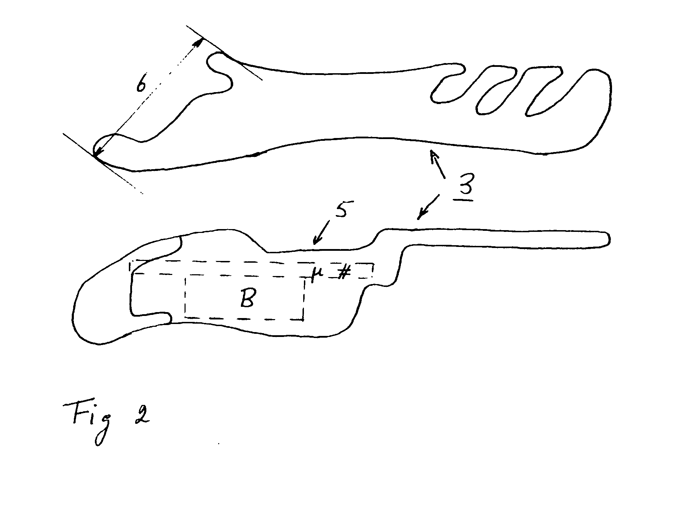

Figure 1 is a very schematic view of an information-processing and monitoring system 1, called information system hereinafter. In one variant, the system 1 is suitable for practically continuous monitoring of the health condition in particular of animals removed from a base station 2 by some distance, and in another variant the system 1 is suitable for taking action by remote control, continuously if necessary, depending on the diagnosis made, for treating the disorder or impending disorder of the animal present in the field or in the stable. Every animal included in the system 1 is provided with a unit 3, which, apart from a local, usually programmable processor provided with a storage memory # for controlling and computing, is provided with more than one sensor S1, S2 ... Sn connected thereto (only shown in figure 1) and a transmitter 4 that can be activated by the control unit, which transmitter is capable of communicating regularly, practically continuously, information, for example every few minutes, wirelessly to the base station 2 in a reliable manner, synchronously or asynchronously, according to a desired protocol and format. The interval may be determined by the local control unit, but also by the base station 2, in which case each unit in the ear, hereinafter called ear unit 3, will also comprise a receiver, thus forming a transceiver 4. The ear unit 3 may be provided with or at least communicate with an RFID unit (not shown) disposed somewhere on or in the animal's body so as to enable further communication with process computers, a GPS receiver or location engine for determining the location and/or the elevation. A loudspeaker may be provided for producing sound.
Furthermore, LEDs may be provided for visualising inter alia the setting or status of one or more sensors, or, for example, giving a visual indication of an alarm during the time that the sensor is operative or for visualising an emergency situation.
The unit 3 is fed by a - possibly rechargeable - battery B (shown in figure 2) and/or by a solar cell that may be connected thereto. An important part of the locally available electric power is consumed during transmission by the transmitter 4. The battery 4 may also be charged by using a mechanism which converts kinetic energy and/or thermal energy and/or a difference temperature into electric energy. Because the transmitter 4 is on only briefly after being activated, each ear unit 3 consumes only little electric energy.

The sensors S are capable of continuously measuring the most widely varying quantities of each individual animal. Examples of such quantities are temperature, sound, activity, ruminating activity, movement, heart rate, breathing, biochemical values and the like. In addition to temperature sensors and a sound sensor for detecting sounds in various frequency ranges, it is also possible to use a 3D sensor (accelerometer) for overall measurement of the movements made by the animal. Furthermore, quantities can be measured and transmitted by external sensors outside the ear. Local computations may be made for determining important parameters or statistical quantities or for carrying out some kind of statistical pre-analysis.

If the measurement results are found to exhibit an abnormality, and a first analysis result as regards the kind of disorder is available, if possible, the base station 2, after having received the data from a respective ear unit 3, can send a message, e-mail or SMS message over the Internet to the cattle farmer's PC or, for example, to his PDA or telephone, informing him that the animal or animals in question must receive attention. These types of transmission options and other options, such as the general control, and the presentation and availability of information is controlled by a master (indicated AGIS).

In one variant, the ear unit 3 is for example arranged for activating the transmitter 4 when the local measurement shows that there are individual measurement data that fall outside the normal range of the animal in question or of the standard animal group, for example. Setting aside the transmission of signals by an ear unit 3 to the base station 2 to indicate that everything is functioning correctly, which is usually desirable, a strongly reduced local consumption of electrical energy by the transmitter 4 is obtained in this way, because the majority of the animals are healthy and there is no need to transfer data to the base station 2.

From the measurement of the aforesaid complex of transmitted vital signals and quantities, important conclusions can be drawn for each individual animal, and also for the group of animals as a whole. This takes place mainly in the base station 2. Illnesses or disorders that can be diagnosed at an early stage on the basis of the measured values and the changes in the measurement data, among which the temperature, include: the presumed time of calving, the occurrence of uteritis, inflammation of the udder, the period that the animal is in heat, whitlow, Mastitis, Mortellaro, blue tongue, contamination with viruses or bacteria such as salmonella, E-coli or the like, food- or digestion-related disorders, and many other abnormalities that have consequences for the animal's well-being, milk production and meat quality. Furthermore, a quicker detection of contagious diseases, such as foot-and-mouth disease or the like, can be realised.

If the animals can spread over larger distances in the field, a respective transmitter or transceiver 4 still does not need to have a larger transmission range if one or more intermediate ear units are capable of relaying the signal from and/or to the remote animal, and only need to forward said signal to a next relaying unit, therefore, and eventually to the base station or the unit of the remote animal. This leads to an even further reduction of the local energy consumption in each ear unit, because the required power level for the final stage of the transmitter increases as the transmission range increases. Some units 3 may temporarily function as a relaying unit, if desired, for example when such a unit detects that a remote unit is attempting to transmit something at some point in time, which attempts have so far not been successful. Furthermore, the base station can inform a selected ear unit 3, if necessary, that it must temporarily function as a relaying unit, for example when there have been no communications from a unit for some time.

An ear unit 3 as shown in the form of two partial views in figure 2, comprises more than one sensor S disposed at two or more different places in the ear canal for measuring the same parameter at more than one position or depth in the ear. In such a dual case, the sensor S1 corresponds to sensor S2, for example, which is disposed slightly deeper, and S1 and S2 both measure the temperature at that depth in the ear canal. This arrangement is used with a view to obtaining the advantages of an improved and earlier diagnosis and analysis as described above.

In one embodiment of the ear unit 3, by means of which it is even possible to treat a disorder, the unit comprises a cavity 5 which is open towards the animal's ear canal and/or is porous, which cavity can be filled with a medicinal and/or health-promoting substance, which is subsequently gradually released therefrom for a prolonged period of time, without any further human interference by the cattle farmer, to the blood vessels in the ear canal and thus to the animal's blood. This has been found to be very advantageous in the case of some disorders, which need to be treated for a prolonged period of time. The porous cavity may have been made porous by using a slightly open gauze or fabric with a suitable mesh width. The cavity 5 may also be completely open towards the ear cavity, as is shown in the figure.

In an advanced variant thereof, the system 1 is arranged for regulating, from the base station 2, the amount of substance being released into the blood vessels of the ear canal. This may for example be done by shutting off the cavity 5 by means of a controllable regulator, so that the amount of substance being released can be influenced either locally on the basis of the currently measured parameters or on the basis of guidelines or instructions from the base station 2. It can also be done by configuring the cavity 5 as a multi-part cavity and opening the various parts, which may be filled with different substances, at the command of the base station 2, for example.

The ear unit is preferably configured such that additional rigidity is imparted to the slightly wedge-shaped rib 6 of the ear unit 3 that is to be provided between helix and concha in the ear. The unit 3 hooks in the animal's ear canal, as it were, in that case and cannot become detached therefrom upon movement of the animal's head. The ear unit is additionally secured by connecting the right-hand part of the ear unit 3 shown in figure 2 to the animal's ear tag that is already present. The required stiffness of said intermediate part ranges between 35 - 75 shore, in particular between 50 - 70 shore, more in particular between 60 - 65 shore. The remaining part of the hollow ear unit 3 may be fabricated of a flexible plastic.

The system 1, which has been described in relation to animals in the foregoing, may also be used with people, but also with children, for example babies.

In practice it is advantageous for a smooth insertion and wearing of the ear unit 3 to apply an at least partly stiffness of the plastic of the unit 3 of around 35-45 shore, more specifically around 40 shore. If to flexible the unit 3 may give way to easily, which consequently leads to an inadvertent loosing of the unit 3 in case the person or animal moves its head in a normal way. If too inflexible the unit 3 may be difficult to insert or wear, in particular because animals such as cows appear to have a tendency and desire to change the form and/or position of their auditory canal and/or ear, which is not an abnormal phenomena.

The above described measurement of the mentioned quantities, i.e. temperature(s) or the temperature difference(s) at various depth positions within the ear canal can of course take place in the same ear, with only one ear unit 3 being required carrying several sensors, or at wish with two units 3 which are then included in ear units 3 present in both ears.

Quantities that can also be measured as biochemic values either continuously or discontinuously are the oxygen level or the sugar level in the blood circulating in and around the ear canal. Such oxygen measurements or saccharimetry respectively may take place by means of appropriate sensors, in particular optic sensors and by means of electronics capable of deriving the respective values from the data provided by the sensor, in order to use that value for an overall determination of the medical condition of the person, adult, child or animal concerned.

## Claims

1. An information system (1) for use with animals, comprising a base station (2) and a number of units (3) being in wireless communication with said base station (2), which units (3) are to be placed in the animal's ear and which are provided with one or more sensors (S1, S2 ... Sn) for periodically measuring quantities such as temperature, sound, (ruminating) activity, movement, heart rate and/or biochemical values, at least in the ear, and which are connected to an activable transmitter (4) for transmitting, upon activation of said transmitter (4), individual sensor measurement data to the base station (2),
**characterised in that** the ear unit (3) is provided with more than one sensor (S1, S2) capable of measuring the temperature and disposed at two or more depths in the ear canal for measuring the same quantity at several depths in the ear.

2. A system (1) according to claim 1, **characterised in that** the ear unit (3) is provided with a processor which is arranged for activating the transmitter (4)) when a measurement establishes the existence of individual sensor measurement data that fall outside a normal range.

3. A system (1) according to claim 1 or 2, **characterised in that** the base station (2) and the ear units (3) are connected to or provided with transceivers (4).

4. A system (1) according to claim 3, **characterised in that** one or more of the transceivers are configured as a relaying unit (4).

5. A system (1) according to any one of claims 1-4, **characterised in that** the ear unit (3) comprises a cavity (5) which is open towards the ear canal or which is porous, which cavity can be filled with a medicinal and/or health-promoting substance.

6. A system (1) according to claim 5, **characterised in that** the system (1) is arranged for influencing the amount of substance being released in blood vessels of the ear canal from the base station (2), for example by regulating or opening one or more closed parts of the cavity (5).

7. An ear unit (3) suitable for use in the information system (1) according to any one of claims 1-6, **characterised in that** the ear unit (3) has a certain degree of stiffness between the parts of the ear unit (3) to be provided between helix and concha in the ear.

8. An ear unit (3) according to claim 7, **characterised in that** said stiffness ranges between shore 35 and 75, in particular between shore 35-45, more in particular around shore 40.

## Patentansprüche

1. Informationssystem (1) für den Einsatz mit Tieren, umfassend eine Basisstation (2) und eine Anzahl von Einheiten (3), welche sich in drahtloser Verbindung mit der Basisstation (2) befinden, wobei die Einheiten (3) in den Ohren der Tiere angeordnet sein müssen und mit einem oder mehreren Sensoren (S1, S2 ... Sn) zum periodischen Messen von Messgrößendaten wie Temperatur, Geräusche, (Wiederkäu-) Aktivitäten, Bewegung, Herzschlagfrequenz und/oder biochemische Werte, wenigstens im Ohr, ausgestattet sind und wobei sie mit einem aktivierbaren Sender (4) zum Übertragen bei Aktivierung des Senders (4) einzelner Sensorenmessdaten an die Basisstation (2) verbunden sind,
**dadurch gekennzeichnet, dass** die Ohreinheit (3) mit mehr als einem Sensor (S1, S2) ausgestattet ist, welche in der Lage sind, die Temperatur zu messen, und an zwei oder mehreren Tiefen im Ohrkanal zum Messen derselben Messgröße an mehreren Tiefen im Ohr angeordnet sind.

2. System (1) nach Anspruch 1, **dadurch gekennzeichnet, dass** die Ohreinheit (3) mit einem Prozessor ausgestattet ist, welcher für das Aktivieren des Senders (4) ausgelegt ist, wenn eine Messung das Vorhandensein einzelner Sensorenmessgrößendaten ergibt, die außerhalb eines normalen Bereichs fallen.

3. System (1) nach Anspruch 1 oder 2, **dadurch gekennzeichnet, dass** die Basisstation (2) und die Ohreinheiten (3) mit kombinierten Sende- und Empfangsvorrichtungen (4) verbunden sind.

4. System (1) nach Anspruch 3, **dadurch gekennzeichnet, dass** eine oder mehrere der Sende- und Empfangsvorrichtungen zu einer Vermittlungseinheit (4) konfiguriert ist/sind.

5. System (1) nach jedem beliebigen der Ansprüche 1 - 4, **dadurch gekennzeichnet, dass** die Ohreinheit (3) eine Höhle (5) umfasst, welcher in Richtung des Gehörgangs offen ist, oder welche porös ist, wobei die Höhle mit einer medizinischen und/oder gesundheitsförderlichen Substanz gefüllt sein kann.

6. System (1) nach Anspruch 5, **dadurch gekennzeichnet, dass** das System (1) zur Beeinflussung der Menge an Substanz, welche in die Blutgefäße des Gehörgangs freigesetzt wird, durch die Basisstation (2) angeordnet ist, zum Beispiel durch Regeln oder Öffnen einer oder mehrerer geschlossener Teile der Höhle (5).

7. Ohreinheit (3), geeignet für den Einsatz im Informationssystem (1) nach jedem beliebigen der Ansprüche 1 - 6, **dadurch gekennzeichnet, dass** die Ohreinheit (3) einen gewissen Grad an Steifigkeit zwischen den Teilen der Ohreinheit (3) aufweist, welche zwischen Helix und Concha im Ohr eingesetzt werden müssen.

8. Ohreinheit (3) nach Anspruch 7, **dadurch gekennzeichnet, dass** die Steifigkeit zwischen Shorehärte 35 und 75, insbesondere zwischen Shorehärte 35-45, und im Besonderen um Shorehärte 40 liegt.

## Revendications

1. Système d'information (1) destiné à être utilisé avec des animaux, comprenant une station de base (2) et un nombre d'unités (3) se trouvant en communication non filaire avec ladite station de base (2), les unités (3) étant à placer dans l'oreille de l'animal et pourvues d'un ou de plusieurs détecteurs (S1, S2... Sn) pour mesurer périodiquement des quantités telles que la température, le son, l'activité (rumination), le mouvement, le rythme cardiaque et/ou les valeurs biochimiques, au moins dans l'oreille, et étant reliées à un transmetteur actionnable (4) pour la transmission de données de mesure des détecteurs individuels à la station de base (2) lors de l'activation dudit transmetteur (4),
**caractérisé en ce que** l'unité d'oreille (3) est pourvue de plus d'un détecteur (S1, S2) capable de mesurer la température et disposé à deux ou plusieurs profondeurs dans le canal auditif, afin de mesurer la même quantité à plusieurs profondeurs dans l'oreille.

2. Système (1) selon la revendication 1, **caractérisé en ce que** l'unité d'oreille (3) est pourvue d'un processeur conçu pour l'activation du transmetteur (4) lorsqu'une mesure établit l'existence de données de mesure de détecteurs individuels non incluses dans une plage normale.

3. Système (1) selon la revendication 1 ou 2, **caractérisé en ce que** la station de base (2) et les unités d'oreille (3) sont pourvues d'émetteurs-récepteurs (4) ou connectées à ceux-ci.

4. Système (1) selon la revendication 3, **caractérisé en ce qu'**un ou plusieurs émetteurs-récepteurs sont configurés comme des unités de relais (4).

5. Système (1) selon l'une quelconque des revendications 1 à 4, **caractérisé en ce que** l'unité d'oreille (3) comprend une cavité (5) ouverte vers le canal auditif ou poreuse, la cavité pouvant être remplie avec une substance médicale et/ou de promotion de la santé.

6. Système (1) selon la revendication 5, **caractérisé en ce que** le système (1) est conçu pour influencer la quantité de substances diffusées dans les vaisseaux sanguins du canal auditif à partir de la station de base (2), par exemple en régulant ou en ouvrant une ou plusieurs parties fermées de la cavité (5).

7. Unité d'oreille (3) adaptée pour une utilisation dans le système d'information (1) selon l'une quelconque des revendications 1 à 6, **caractérisée en ce que** l'unité d'oreille (3) présente un certain degré de rigidité entre les parties de l'unité d'oreille (3) à disposer entre l'hélix et le conque dans l'oreille.

8. Unité d'oreille (3) selon la revendication 7, **caractérisée en ce que** ladite rigidité varie entre 35 et 75 shore, en particulier entre 35 et 45 shore, et plus particulièrement autour de 40 shore.
